# EUROPEAN PATENT SPECIFICATION

(11) **EP 1 052 935 B1**
(45) Date of publication and mention of the grant of the patent: **30.07.2008**
(21) Application number: 99906698.8
(22) Date of filing: 01.02.1999
(51) Int. Cl.: A61B 5/06, A61M 5/142

(54) **SYSTEM FOR LOCATING IMPLANTABLE MEDICAL DEVICE**
ANORDNUNG ZUR ORTUNG EINER IMPLANTIERBAREN MEDIZINISCHEN VORRICHTUNG
SYSTEME DE LOCALISATION DE DISPOSITIF MEDICAL IMPLANTABLE

(30) Priority: 02.02.1998 US 17198
(43) Date of publication of application: 22.11.2000
(73) Proprietor: MEDTRONIC, INC., Minneapolis, Minnesota 55432-5604 (US)
(72) Inventor: WEIJAND, Koen, J., NL-3235 EG Rockanje (NL); HALLER, Markus, CH-1268 Begnins (CH)
(74) Representative: Hughes, Andrea Michelle
(86) International application number: PCT/US1999/002173
(87) International publication number: WO 1999/038438

(56) References cited:
- WO-A-96/32060
- DE-A- 4 037 586
- DE-B- 1 100 874
- US-A- 5 211 165
- US-A- 5 375 596
- US-A- 5 443 066

## Description

The present invention relates to implantable medical devices and, particularly, to an implantable medical device system for locating an implantable medical device with a high degree of precision.

Many implantable medical devices require percutaneous communication. That is, in particular regard to implantable drug infusion devices, such devices often require the drug supply to be replenished. Typically, such replenishment is accomplished by inserting a needle through the skin and into the septum of a drug reservoir in such a device.

Because such a device is implanted and thus not able to be directly seen, care must be taken to ensure that the needle is properly placed into the device before injection. If the needle misses the device and, in particular, misses the drug reservoir in the device, the drugs will be immediately dispensed in the body, having potentially dire consequences for the patient. Moreover, if the needle is not fully placed through the septum and into the drug reservoir, the drug reservoir will not be adequately filled, also having potentially dire consequences for the patient.

Previous attempts have been made to accurately locate and identify implanted devices and, in particular, septum loading to the drug reservoir of implantable drug infusion devices. For example, Celcontrol, Inc. advertised an implantable vascular access device which required the attachment of an electrode to the skin and the attachment of a wire to the hypodermic needle to create a circuit for locating the implantable device. Such a system, besides having more complexity than desired, did not provide an accurate location of the needle in relation to the device without first inserting the needle through the skin. U.S. 5,211,165 describes a tracking system to follow the position and orientation of a device with radiofrequency field gradients. DE-B-1100874 describes medical examination equipment using radio signals. DE-A-4037586 describes a medical probe carrying a transmitter or receiver aerial in its tip. US-A-5,433,066 describes an invasive imaging system employing a radiofrequency tracking system. U.S. Patent 5,171,228 disclosed a further system which required an RF transmitter and a transmitting antenna. Such a system, to date, has not proven practical or provided an acceptable precision for locating the implantable device. Thus, there exists a need for a simple device and technique for sensing the position of an implanted device and, in particular, of a drug reservoir septum, without first requiring the skin to be punctured or additional electrode attachments to be made to the patient.

These and other objects are accomplished by the present invention which includes a system for locating an implantable medical device. The invention provides a system for locating an implantable device comprising:
an implantable device,
a transmitter;
an array of antennas, the array having a first receiving antenna and a second receiving antenna, a processor coupled to the antennas, the processor measuring the amount of energy sensed by each of the antennas from the transmitter; either the transmitter or the array being integrated with said implantable device, and further **characterized by** the processor having an equality sensor for sensing whether the energy sensed by all of the antennas are equal.

The system senses the proximity to the implant coil and, thus, the implant device by determining when an equal amount of energy is present in each of the antennas of the antenna array and if each such ducted energy is greater than a predetermined minimum. When such a condition is met, the antenna array is aligned with the implant coil. Thus the needle port through the antenna array is lined up with the septum of the drug reservoir. Alternative embodiments are further disclosed in which the processor and antenna array are positioned within the implanted device while the coil is external to the patient.

Preferred embodiments will now be described, by way of example only, with reference to the accompanying drawings.
FIG. 1 is a block diagram showing a system to the present invention.
FIGS. 2A and 2B are a top view of an antenna array.
FIG. 3A is a top view of an implant coil.
FIG. 3B depicts the implant coil positioned coaxial with the septum of device.
FIG. 3C depicts an alternative configuration of implant coils.
FIG. 3D depicts an alternative configuration of the antenna array.
FIG. 4 discloses a circuit for use in the location processor.
FIG. 5 discloses a procedure used to locate a device according to the present invention.
FIG. 6 discloses an alternative circuit for use in the location processor.
FIG. 7 is a block diagram showing an alternative system according to the present invention.
FIGS. 8 and 9 depict an alternative embodiment of the present invention.
FIG 10 shows the measured RSSI signal when septum locator is off centered.
FIG. 11 is a further alternative embodiment of the present invention.
The FIGS. are not necessarily to scale.

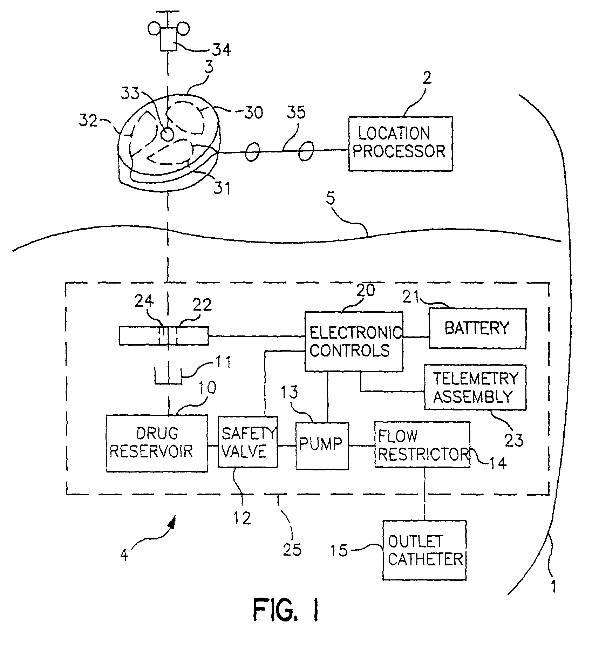

FIG. 1 is a block diagram showing the system according to the present invention. As seen, the system 1 features a location processor 2 coupled to implant location antenna array 3 which are used to locate implantable medical device 4 positioned beneath the skin of patient 5. Medical device 4 may be of any design desired, such as the Medtronic SynchroMed^{®} implantable drug pump, although other devices besides drug pumps may also be used. In the preferred embodiment device 4 includes a drug reservoir 10 having a septum 11 for drug replenishment. The drug reservoir outlets such drugs, past pump 13 to outlet catheter 15. In an additional embodiment (although illustrated here for clarity) the device could further feature a safety valve 12 and , beyond flow restrictor 14, constructed according to the PCT patent application WO99/38551 entitled "Implantable Drug Infusion Device Having A Safety Valve" of Markus Haller and Koen Weijand and PCT patent application WO99/38552 entitled "Implantable Drug Infusion Device Having A Flow Regulator" of Markus Haller, Phillipe Renaud and Christian Amacker respectively both filed on 2 February 1998 and published on 5th August 1999. Electronic controls 20 powered by battery 21 provide control and energy to the pump and the safety valve and further provide control and energy to implant coil 22. The device further includes a telemetry assembly 23 to provide two-way communication between device 4 and any suitable external device. As seen, implant coil 22 is positioned such that opening 24 therein is aligned with septum 11. The coil is aligned with an opening centered therein. In this view the implant coil is shown positioned above the septum, although the implant coil may also be positioned around or, indeed, beneath the septum. What is important is for the coil and thus the opening and the septum to be aligned. As further seen, all elements of the device but the outlet catheter are housed within the hermetic enclosure 25 as is well known in the art.

As further seen in this figure, implant location antenna array 3 is movably positioned outside of patient 5. The array features three air coil antennas 30, 31 and 32 symmetrically disposed about the guide 33 and all mounted within the same plane. As seen, guide 33 is provided to permit a needle 34 to be positioned through the array and thus into and past septum 11 to thereby replenish drugs in the reservoir. Each antenna is electrically coupled to the location processor through a series of wires, commonly designated 35.

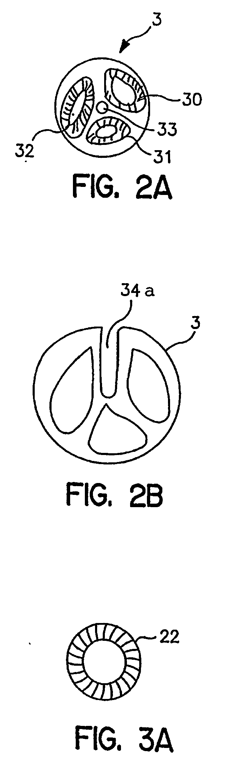

Turning now to FIG. 2A which shows a top view of array 3, as seen, antennas 30, 31 and 32 are symmetrically disposed about guide 33. The antennas, moreover, are also disposed along the same plane. Each antenna is identical, and is constructed as a planar air coil antenna, preferably from a printed circuit board, although other antenna constructions may be used, such as a ferrite coil. The planar antenna is housed within a non-conductive material, such as plastic. The guide 33 is disposed in a center location and is sized to permit a needle to be passed therethrough. Although shown as a circular passage, a needle slot 34a may further be provided, as shown in FIG. 2B. As discussed above, the antenna is positioned external to the patient and is designed to be moved along the surface of the patient's body to thereby accurately locate the implant coil and thus the septum of the device.

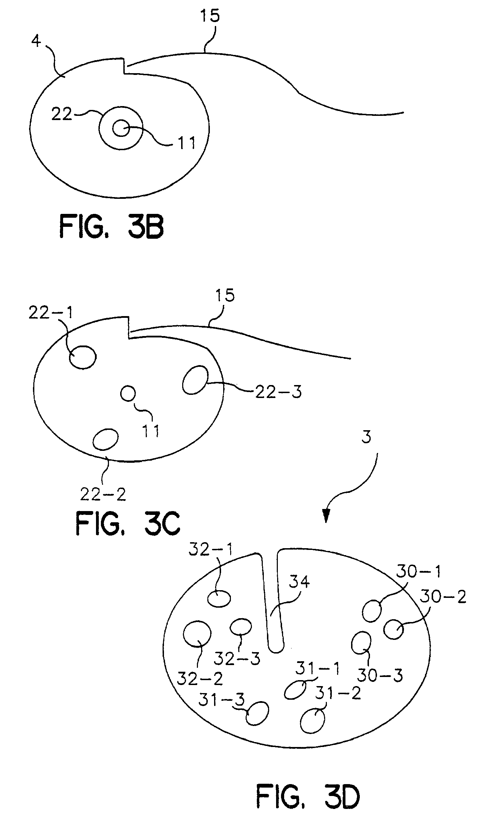

FIG. 3A is a top view of implant coil 22. The implant coil is constructed of any acceptable material. Although shown in FIG. 1 as a separate coil and device it should be appreciated that the implant coil could also be fashioned by using an implant telemetry of the device as is well known to one skilled in the art. FIG. 3B depicts the implant coil 22 positioned coaxial with the septum 11 of device 4. FIG. 3C depicts a device 4 which features an alternative configuration of coil. In particular in this embodiment a series of coils 22-1, 22-2 and 22-3 are used. Such plurality of coils may be used to each emit at differing frequencies so that the array not only may accurately sense the septum's location, but also the proper orientation of the array to the device may also be detected. As seen in FIG. 3D in this embodiment the array 3 would feature a series of matching sensing arrays 30-1, 30-2, 30-3; 31-1, 31-2, 31-3; 32-1, 32-2, 32-3 of antennas, each series attuned to sense the output of the corresponding coil. While this embodiment is a bit more complicated than that shown above, it provides the same function without having to locate the coil around the septum.

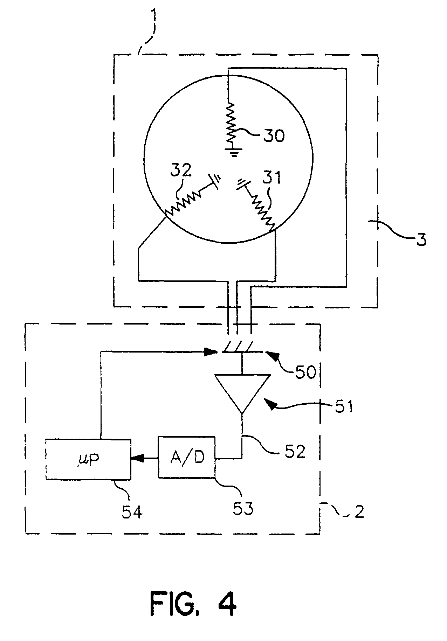

FIG. 4 discloses the circuit used in the location processor 2 and array 3. As seen, antennas 30, 31 and 32 disposed in array 3 are coupled into location processor 2 through switch 50. Through such a coupling this embodiment uses a sampling technique to alternatingly sample the signal on each antenna. Each such sampled signal is then passed through amplifier 51 which also provides a filtering function and outputs the signal on line 52 as an RSSI. In the preferred embodiment, amplifier is preferably NE604, available from National Semiconductor Corporation, 2900 Semiconductor Drive, P.O. Box 58090, Santa Clara, California, 95052-8090. The signal is then processed through analog digital computer 53 where it is then put into the microprocessor 54. The microprocessor thereafter compares each of the signals sampled from the antennas and determines whether the energy received by each of the antennas is above a predetermined minimum. In such a case an operating range signal is emitted to indicate to the operator the operating distance with the implanted device, as discussed in more detail below. The microprocessor would then determine whether the same amount of energy is being sensed by each antenna, which, due to the geometry of implant coil 22 and array 3, indicates the antennas having a guide therein as well as the implant coil are thus in alignment. The microprocessor would then cause to be emitted an alignment signal. As discussed in more detail below, in an alternative embodiment, rather than using a sampling technique to detect the energy sensed by each antenna, the system could also use a technique in which each coil is oppositely coupled , that is in anti-phase, such that when a null is sensed the coils are each sensing an equal amount of energy.

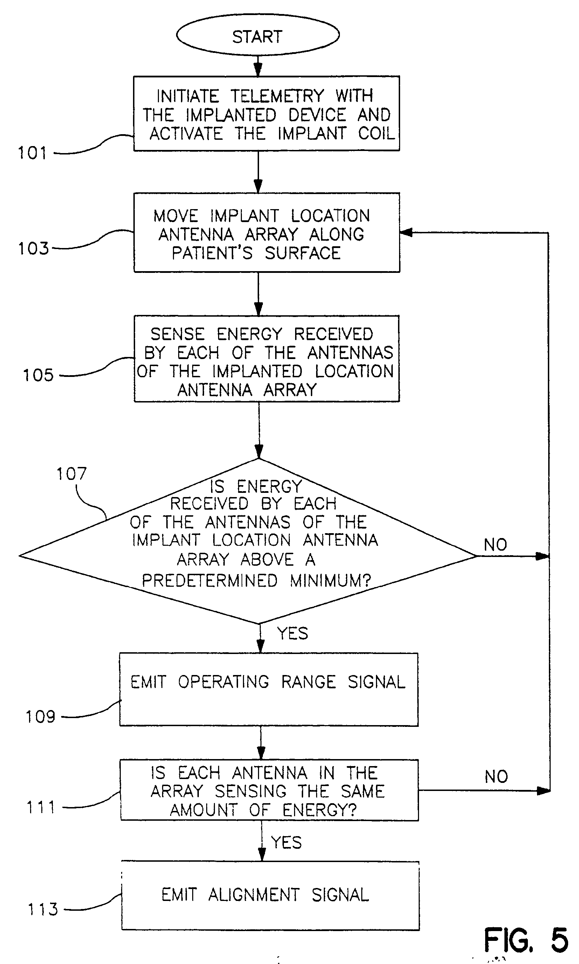

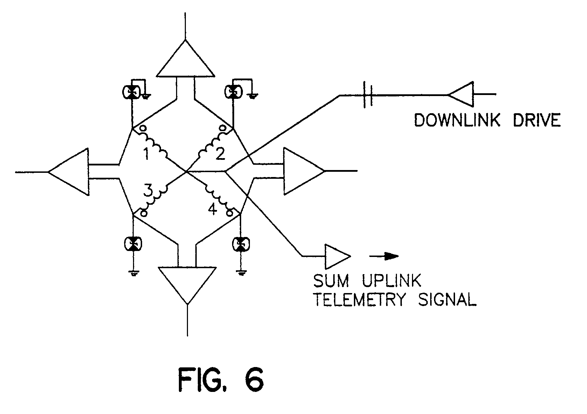

FIG. 5 discloses the procedure used to locate a device according to the present invention. As seen at 101, telemetry is initiated with the implant device. Such telemetry initiation is done to instruct the implant device to activate the implant coil. In the preferred embodiment the implant coil is activated to thereby transmit energy at a known frequency, preferably the device transmits at 32.768 kHz since this frequency is readily available in present implantable pulse generators. Next, at 103 the implant location antenna array is moved along the patient's surface. Next, at 105 the energy received by each of the antennas of the implant location antenna array is sensed. Next, at 107 the device determines if such energy is above a predetermined minimum. As can be appreciated, this predetermined minimum amount of energy required to be sensed by each antenna dictates the furthest distance the antenna of the array may be from the implant coil, and still provide information regarding the location of the coil and thus the device. Although determining whether the energy sensed by each antenna is above a predetermined threshold is shown as a separate step, this could also be integrated within another step. Next, if the energy sensed is above a predetermined minimum the device goes to step 109 and emits an operating range signal, otherwise the device recycles back to step 103. Next, at 111 a comparison is made to determine if each of the antennas in the array is sensing the same amount of energy. Comparison may be made using a sampling technique, as described above in FIG. 4, or a non-sampling technique, as described below with regard to FIG. 6. With either or any other technique, if it is found that each of the antennas is sensing an equal amount of energy, then If the antennas each are sensing the same amount of energy which is above the predetermined minimum, then this implies each of the antennas is positioned an equal distance away from the implant coil. Because the passage and septum are located directly centered within the array and the coil respectively, this then means that they are in alignment. Next, at 113 a signal is emitted by the location processor to indicate such alignment. The emitted signal is preferably a light signal, although this may also be delivered through a sound or both. In a further embodiment the array or the device may additionally feature a vibration device, which is either activated or deactivated (whichever is desired) upon alignment. Through such a manner the transmission of energy from the coil to the array may be used to precisely align the components.

FIG. 6 discloses an alternative circuit for use in the location processor 2. As seen in this embodiment four separate antennas are used in the array. Each coil is further connected to the associated electronics such that the voltages from the coils are subtracted. The center connection of the coils provides the sum of the signals sensed by all the coils. Because the signal from two coils connected in anti-phase is only zero, the symmetry line of these coils crosses the center of the transmitting antenna, in this case the implant coils 22. This means that all four signals derived are zero, the four symmetry lines of these antennas are crossing the center of the implant coil. In addition, the sum signal must be the maximum, or at least greater than the predetermined value, thereby indicating the antenna is in connection with an implant coil. As already discussed above, this determines that the device is within a predetermined operating range. Although shown as having four antennas, this embodiment may further be constructed using any number of antennas, from 3 to 360, for example. Among the advantages believed offered by such a configuration is the ability to combine both device telemetry and localization.

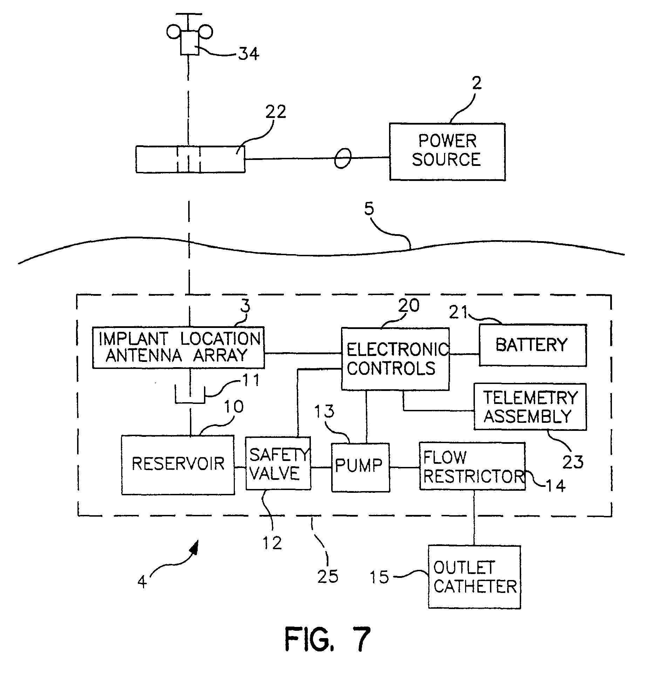

FIG. 7 is a block diagram showing an alternative system according to the present invention. As seen this system differs from that disclosed in FIG. 1 in that the location processor 2 coupled to implant location antenna array 3 is integrated within the device 4 while the coil 22 is featured outside of the patient. Like the system of FIG. 1, device 4 may still be of any design desired. The device may include a drug reservoir 10 having a septum 11 for drug replenishment. The drug reservoir outlets such drugs through safety valve 12, past pump 13, beyond flow restrictor 14 to outlet catheter 15. Electronic controls 20 powered by battery 21 provide control and energy to the pump and safety valve and further provide control and energy to implant coil 22. Device further includes a telemetry assembly 23 to provide two-way communication between device 4 and any suitable external device. In this embodiment, array 3 (rather than implant coil 22 as shown in FIG. 1) is positioned such that a guide 33 therein is aligned with septum 11. In this view array 3 is shown positioned above septum, although array 3 may also be positioned around or, indeed, beneath the septum. As already discussed above, what is important is for array 3 and in particular guide 33 and septum to be aligned, As further seen, all elements of the device but the outlet catheter are housed within the hermetic enclosure 25 as is well known in the art. As further seen, coil 22 is movably positioned outside of patient 5. Coil 22 is of the same design discussed above, and features an opening 24 (either a slot or a hole) therein to permit a needle 34 to be positioned through array and thus into and past septum 11 to thereby replenish drugs in the reservoir. In a still further embodiment the syringe can be constructed having an integral coil.

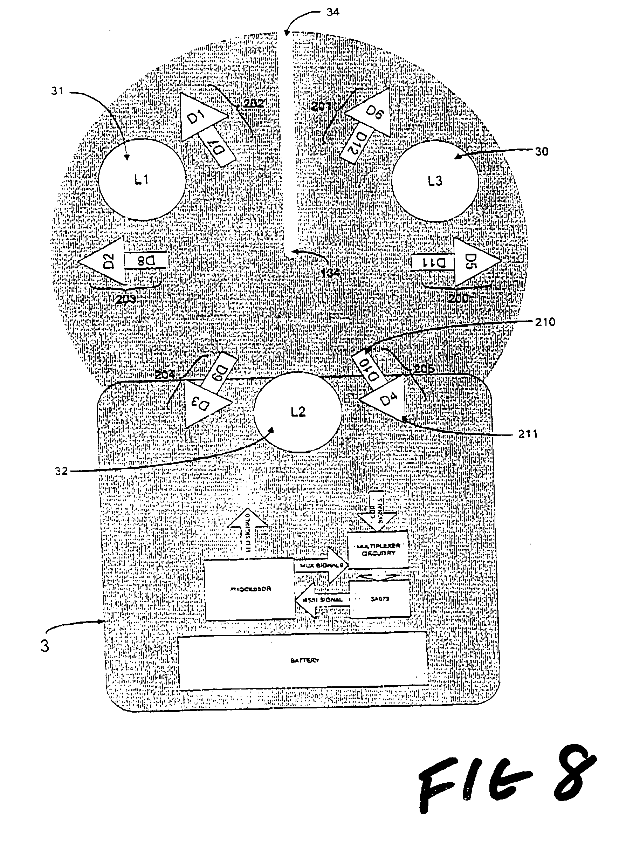

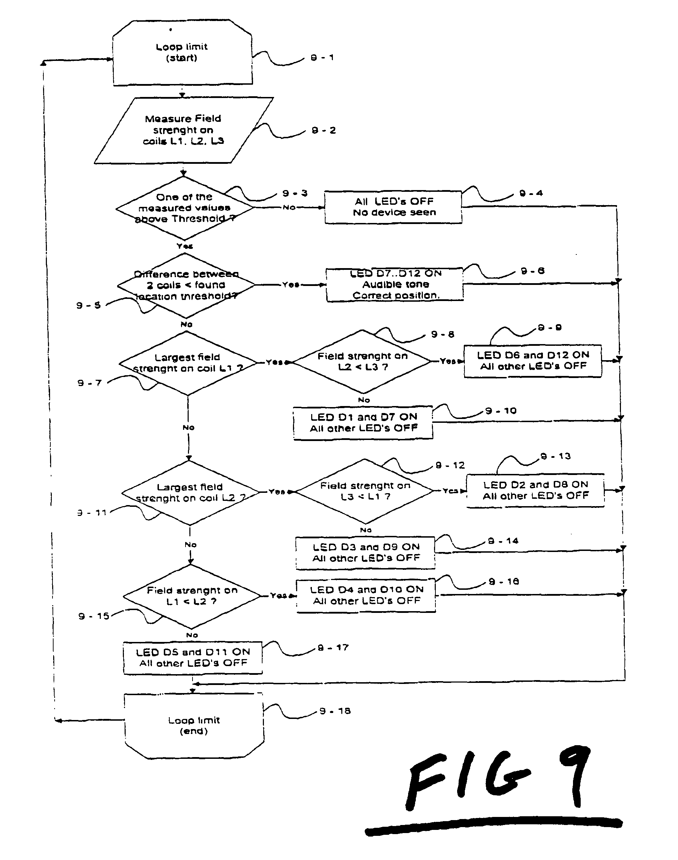

FIGS. 8 and 9 depict an alternative embodiment of the present invention, in particular, an embodiment featuring a guiding mechanism system for locating implantable medical device. As already discussed above, the present invention permits the locating implantable medical device through the sensing of an equal amount of energy on each of the antennas of the antenna array. When such an equal amount of energy is sensed, then the external locator and the implant coil are aligned. This additional alternative embodiment includes a method and apparatus which features a guiding mechanism system for locating implantable medical device. In particular, the sensed energy on each of the antennas of the antenna array is used as the input parameters for a routine which determines the way the user has to move the external locator in order to align with the implanted medical device. The direction in which to move the external device may be given by visual and/or audible indication to the user. In the preferred embodiment this is done by illuminating a tail and an arrow LED to point to the direction to move the locator. Whenever the correct position is found, only the tail LEDs are illuminated. A possible mechanical setup is shown in FIG. 8. A flowchart for the guiding routine can be found in FIG. 9.

FIG. 8 depicts a mechanical set up for septum locating array 3 according to the alternative embodiment. As seen in this embodiment, the array includes a guide slot 34 which, as discussed above, reaches into a midpoint 134 between the various sensing coils, 30, 31, 32.

In this embodiment, the array further features a series of directional indication signals 200-205. Referring to 205, in particular, each signal includes both a tail portion 210 and a head portion 211. Head and tail portions are selectively illuminated to provide information to the user in which direction the array should be moved. Thus, when D5 and D11, in the present embodiment, are illuminated, the array should be moved to the right. Once center portion 134 is properly positioned above the septum, and each of the coils senses an equal amount of energy as described above, signals would be illuminated such that only the tail portions would be lit. Thus, head and tail portions are only illuminated when movement is required; only tail portions are illuminated when no movement is required to properly locate center portion 134 relative to the septum. As discussed above, although illumination of such signals is preferred, for this alternative embodiment other indications may also be provided, including audible outputs or, in fact, tangible outputs, such as vibration.

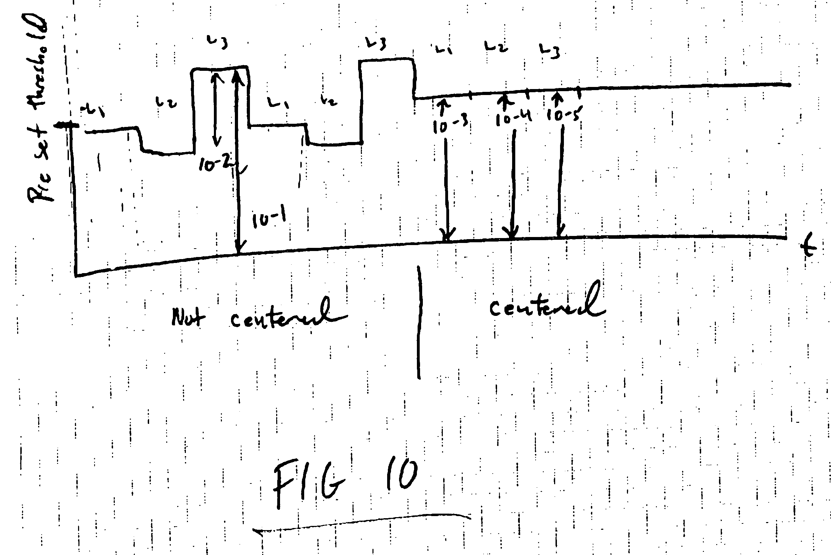

FIG. 9 is a flow chart which illustrates a guiding routine for the illumination of the signals depicted in FIG. 8. As seen at 9-1, the routine is begun and the field strength in coils L1 (31), L2 (32) and L3 (30) is carried out at 9-2. At 9-3 a determination is made whether one of the measured values is above the preset threshold (see 2 in FIG 10). If such threshold is not met, then the device proceeds to 9-4 where all LED signals (in the preferred embodiment) are kept turned off indicating that no device is seen.

If a measured value is above the predetermined threshold, then the routine drops to 9-5 once the difference between two coils is measured. If this measured difference is less than the pre-selected found location threshold (see 1 in FIG 10), then the routine proceeds to block 9-6 where only the tail portions of each of the signals is turned on and all other portions of the signals, such as the head, are turned off thereby indicating a direct position has been found. If permitted, at this stage, an audible tone or tactile response may also be communicated to the user. If the difference between two coils is greater than the found location threshold in block 9-5, then the device proceeds to block 9-7. At 9-7 the device determines whether the largest field strength is seen in coil L1(31). If it is, then the device proceeds to block 9-8 and determined whether the field strength in L2 (32) is less than the field strength in coil L3 (30). If yes, then the device proceeds to block 9-9 in which signals D6 and D12 are turned on while all other LEDs are kept off. Through this illumination the user is permitted to further move the device in the direction shown by the illumination 201, referring again to FIG. 8. If the field strength in coil L2 (32) is not less than L3 (30), then the device proceeds to block 9-10 and turns on the signals LEDs D1 and D7 while keeping all other LEDs off. In a similar manner the device uses the steps shown in blocks 9-12, 9-13 and 9-14 if the largest field strength on coil L2 (32) is detected as shown in block 9-11.

Finally, the device proceeds to step 9-15 and determines whether the field strength in L1 (31) is less than L2 (32). If yes, then the device proceeds to 9-16 and illuminates head and tail portions of signal 205. If the field strength in L1(31) is greater or equal than L2 (32), then the device instead proceeds to block 9-17 and turns on head and tail portions of signal 200. Thereafter the device drops down to 9-18 and the routine loops back to the beginning of the loop (step 9-1).

FIG. 10 depicts the measured signals sensed in the RSSI when the antenna array is off-centered from the septum. As discussed already with regards to FIG. 9, among the first processes performed by the device is determine whether any one of the measured values on coils L1, L2 or L3 is above the preset threshold. As shown, the amplitude on coil L3 of FIG. 10 is above the preset threshold. Thus, the block 9-3, referring again to FIG. 9, is satisfied and further analysis of the sensed signal strength may be performed.

In the current illustration this means that an analysis is performed on the difference between any two of the coils. If the difference between any two of the coils is greater than the preset found location threshold, then the device is not centered. This is shown here as difference 10-2, between the coils L3 and L2. Essentially, because this difference is greater than the predefined found location threshold, the coils are not sensing signals at roughly similar strengths. This would indicate the coils are thus not centered around the transmission coil, positioned above the septum in the preferred embodiment. When the coils L1, L2 and L3 do, however, get put in a position central to the transmitting coil, then this found location threshold corresponding to the difference in signal strength sensed by any of the two coils approaches zero. This is shown also in this figure, namely, in the section in which the amplitude for sections L1, L2 and L3, depicted as lines 10-3, 10-4, 10-5 are almost exactly the same. This indicates these coils are centered about the transmitting coil.

In the preferred embodiment, incidentally, each coil is measured once every 64 ms for a period of no less than 21 ms.

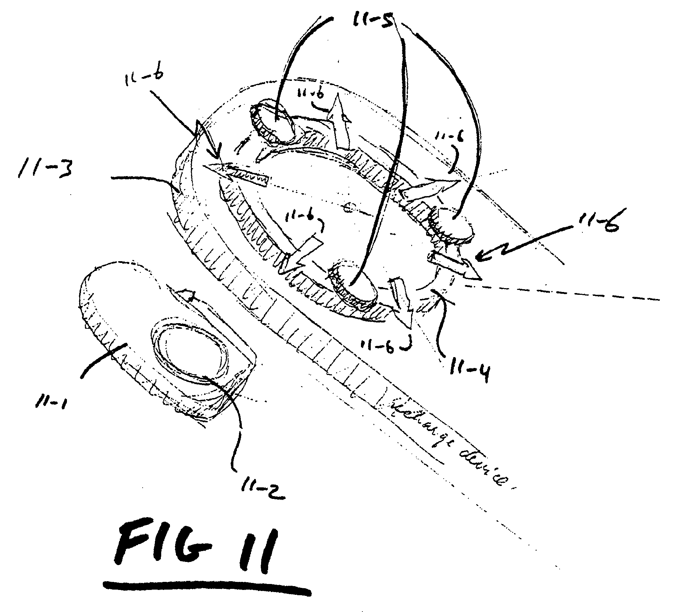

In a still further embodiment, the location detecting system may be further used to accurately locate the optimal position of a recharger to recharge a rechargeable medical device. In such a system, shown in FIG. 11, the IPG 11-1 features a recharge coil 11-2, to receive recharge energy from a recharger. The recharge coil, however, may also be used to first emit a location signal for detection by an array. The recharger 11-3 carries either a separate recharging coil 11-4 and position coils 11-5, which, in turn, are used to activate position indicators 11-6 as described above. Once the optimal position for the device relative to the recharger are determined, the recharging coil 11-4 coil may thereafter be used to deliver recharge energy to the recharge coil and thus to the implanted device. In an alternative design, one or more of the position coils 11-5 may be used in place of a dedicated recharging coil to deliver recharge energy to the IPG. In operation, a rough location can be established by feeling the device. The IPG can start sending the location signal once it has detected that some level of recharge field is present. The external device should alternate its charge to allow reception of the transmitted signal from the IPG and in that way allow for a window to determine the optimal location for the recharge of the device. A magnet can also be used to switch on the signal in the IPG but this assumes that there is still some supply voltage left in the IPG. The location signal does not have to be alternated on and off like the recharge and can be on during the complete recharge cycle. The recharge coil's output for the purpose of signaling device location is triggered through a magnet or by programming initiation.

Although the invention is described as having a separate external positioning array through which a needle is inserted, the invention may also be incorporated as a part of a needle assembly.

## Claims

1. A system for locating an implantable device comprising:
an implantable device (4);
a transmitter (22);
an array of antennas (3), the array having a first receiving antenna (30), a second receiving antenna (31) a third receiving antenna (32), a processor (2) coupled to the antennas, the processor (2) measuring the amount of energy sensed by each of the antennas from the transmitter (22); either the transmitter (22) or the array (3) being integrated with said implantable device, and further **characterized by** the processor (2) having an equality sensor (51, 53, 54) for sensing whether the energy sensed by all of the antennas (30, 31, 32) are equal.

2. A system according to claim 1 wherein the processor (2)intermittently senses the energy received by each antenna (30, 31, 32).

3. A system according to claim 2 wherein the processor (2) intermittently senses the energy received by each antenna for equal amounts of time per antenna.

4. A system according to any preceding claim wherein the processor (2) has a predetermined threshold sensor (51) for sensing whether the energy sensed by each of the antennas exceeds a predetermined threshold.

5. A system according to any preceding claim wherein the array (3) further comprises means (200, 201, 202, 203, 204, 205) for signaling the direction of the transmitter (22) relative to the array (3).

6. A system according to claim 5 wherein the means (200, 201, 202, 203, 204, 205) for signaling the direction of the transmitter (22) relative to the array (3) is coupled to the processor (2), the processor having means for selectively activating the means for signaling.

7. A system according to claim 5 or 6 wherein the means for signaling comprise a series of illuminated arrows (200, 201, 202, 203, 204, 205).

8. A system according to claim 7 wherein the illuminated arrows (200-205) comprise a tail portion and a head portion, the head portion separately illuminatable from the tail portion.

9. A system according to any of the preceding claims wherein the implantable device comprises a drug delivery device (4), the drug delivery device having a drug reservoir (10), the drug reservoir having a drug reservoir septum (11) through which the drug reservoir may be refilled.

10. A method of locating an implanted device (4) by the use of a location system as claimed in any preceding claim, comprising the steps of
emitting energy from the transmitter (22);
moving the antenna array (3) along the patient's surface;
sensing the energy received by each of the antennas of the antenna array; and
emitting, according to the energy sensed by each of the antennas, a device location signal to the array operator.

11. A method of locating an implanted device according to claim 10, further comprising the step of comparing whether the energy received by each of the antennas is the same emitting said device location signal upon sensing that the energy sensed by each of the antennas is the same.

12. A method of locating an implanted device according to claim 10 or 11 wherein the step of emitting a device location signal comprises emitting a light signal.

13. A method of locating an implanted device according to claim 10, 11 or 12 wherein the step of emitting energy from the transmitter (22) is initiated upon the receipt of a telemetry signal by the implanted device (4).

14. A method of locating an implanted device according to any of claims 10 to 13 wherein the step of emitting energy from the transmitter comprises activating an implant coil (22) in the implanted device to transmit energy at a known frequency.

15. A method of locating an implanted device according to any of claims 10 to 14 wherein the step of sensing the energy received by each of the antennas (30, 31, 32) of the antenna array (3) further comprises determining if the energy received by each of the antennas is above a predetermined minimum.

16. A method of locating an implanted device according to claim 15 further comprising the step of emitting an operating range signal according to the result of said determining step.

17. A method of locating an implanted device according to any of claims 10 to 16 wherein the step of emitting a device location signal comprises emitting a sound.

18. A method of locating an implanted device according to any of claims 10 to 17 further comprising the step of providing a tactile output member to the array; wherein the step of emitting a device location signal further comprises altering the output of the tactile output member.

19. A method of locating an implanted device according to claim 18 wherein the step of altering the output of the tactile output member comprises turning the tactile output member off.

20. A method of locating an implanted device according to claim 18 wherein the step of altering the output of the tactile output member comprises turning the tactile output member on.

## Patentansprüche

1. System zum Orten einer implantierbaren Vorrichtung mit:
einer implantierbaren Vorrichtung (4);
einem Transmitter bzw. Sender (22);
einem Antennenarray bzw. -feld (3), wobei das Array eine erste Empfangsantenne bzw. empfangende Antenne (30), eine zweite Empfangsantenne (31), eine dritte Empfangsantenne (32), einen Prozessor (2), der an die Antennen angeschlossen ist, aufweist, wobei der Prozessor (2) die durch jede der Antennen vom Transmitter (22) erfasste Energiemenge misst; wobei entweder der Transmitter (22) oder das Array (3) in bzw. mit der implantierbaren Vorrichtung integriert ist, und ferner **dadurch gekennzeichnet, dass** der Prozessor (2) einen Gleichheitssensor bzw. Übereinstimmungssensor (51, 53, 54) zum Erfassen, ob die die durch alle der Antennen (30, 31, 32) erfasste Energie gleich sind bzw. übereinstimmen, aufweist.

2. System nach Anspruch 1, bei dem der Prozessor (2) die durch jede Antenne (30, 31, 32) erfasste Energie periodisch bzw. intermittierend erfasst.

3. System nach Anspruch 2, bei dem der Prozessor (2) die durch jede Antenne erfasste Energie für gleiche Zeitabschnitte bzw. Zeitintervalle pro Antenne periodisch erfasst.

4. System nach einem der vorstehenden Ansprüche, bei dem der Prozessor (2) einen Sensor zum Erfassen eines vorbetimmten Grenzwertes bzw. einen Vorgabestellwert-Sensor (51) zum Erfassen, ob die durch jede der Antennen erfasste Energie einen vorbestimmten Grenzwert bzw. einen Vorgabeschwellwert übersteigt, aufweist.

5. System nach einem der vorstehenden Ansprüche, bei dem das Array (3) ferner Mittel (200, 201, 202, 203, 204, 205) zum Signalisieren der Richtung des Transmitters (22) in Bezug auf das Array (3) aufweist.

6. System nach Anspruch 5, bei dem die Mittel (200, 201, 202, 203, 204, 205) zum Signalisieren der Richtung des Transmitters (22) in Bezug auf das Array (3) an den Prozessor (2) angeschlossen sind, wobei der Prozessor Mittel zum gezielten Aktivieren der Mittel zum Signalisieren aufweist.

7. System nach Anspruch 5 oder 6, bei dem die Mittel zum Signalisieren eine Reihe leuchtender bzw. illuminierter Pfeile (200, 201, 202, 203, 204, 205) aufweisen.

8. System nach Anspruch 7, bei dem die leuchtenden Pfeile (200 bis 205) einen Schwanzabschnitt bzw. einen Balkenabschnitt und einen Kopfabschnitt aufweisen, wobei der Kopfabschnitt unabhängig vom Schwanzabschnitt beleuchtbar ist.

9. System nach einem der vorstehenden Ansprüche, bei dem die implantierbare Vorrichtung eine Medikamentenabgabevorrichtung (4) aufweist, wobei die Medikamentenabgabevorrichtung ein Medikamentenreservoir (10) aufweist, wobei das Medikamentenreservoir ein Medikamentenreservoirseptum (11) aufweist, durch welches das Medikamentenreservoir wiederbefüllt werden kann.

10. Verfahren zum Orten einer implantierten Vorrichtung (4) durch Benutzung eines Ortungssystems nach einem der vorstehenden Ansprüche, das die Schritte aufweist:
Aussenden bzw. Emittieren von Energie vom Transmitter (22);
Bewegen des Antennenarrays (3) entlang der Oberfläche des Patienten;
Erfassen der durch jede der Antennen des Antennenarrays erfassten Energie; und
Aussenden eines Vorrichtungsortungssignals, das der Energie, die durch jede der Antennen erfasst wird, entspricht, an den Bediener des Arrays.

11. Verfahren zum Orten einer implantierten Vorrichtung nach Anspruch 10, das ferner den Schritt umfasst, zu vergleichen, ob die durch jede der Antennen erfasste Energie gleich ist, wobei das Vorrichtungsortungssignal auf das Erfassen hin, dass die durch jede der Antennen erfasste Energie gleich ist, gesendet wird.

12. Verfahren zum Orten einer implantierten Vorrichtung nach Anspruch 10 oder 11, bei dem der Schritt des Aussendens eines Vorrichtungsortungssignals das Aussenden eines Lichtsignals umfasst.

13. Verfahren zum Orten einer implantierten Vorrichtung nach Anspruch 10, 11 oder 12, bei dem der Schritt des Aussendens von Energie vom Transmitter (22) auf den Empfang eines Telemetriesignals durch die implantierte Vorrichtung (4) hin eingeleitet wird.

14. Verfahren zum Orten einer implantierten Vorrichtung nach einem der Ansprüche 10 bis 13, bei dem der Schritt des Aussendens von Energie vom Transmitter das Aktivieren einer Implantatspule (22) in der implantierten Vorrichtung zum Übertragen von Energie mit einer bekannten Frequenz umfasst.

15. Verfahren zum Orten einer implantierten Vorrichtung nach einem der Ansprüche 10 bis 14, bei dem der Schritt des Erfassens der durch jede der Antennen (30, 31, 32) des Antennenarrays (3) empfangenen Energie ferner umfasst, zu bestimmen, ob die durch jede der Antennen empfangene Energie oberhalb eines vorbestimmten Minimums ist bzw. liegt.

16. Verfahren zum Orten einer implantierten Vorrichtung nach Anspruch 15, das ferner den Schritt des Aussendens eines Bedienungsbereichssignals bzw. eines Betriebsreichweitensignals entsprechend dem Ergebnis des Bestimmungsschrittes umfasst.

17. Verfahren zum Orten einer implantierten Vorrichtung nach einem der Ansprüche 10 bis 16, bei dem der Schritt des Emittierens eines Vorrichtungsortungssignals das Aussenden eines Tons umfasst.

18. Verfahren zum Orten einer implantierten Vorrichtung nach einem der Ansprüche 10 bis 17, das ferner den Schritt umfasst, eine Einheit bzw. ein Element für eine tastbare bzw. taktile Ausgabe bzw. eine Tastausgabeeinheit an das Array bereitzustellen; wobei der Schritt des Aussendens eines Vorrichtungsortungssignals ferner das Ändern der Ausgabe der Tastausgabeeinheit umfasst.

19. Verfahren zum Orten einer implantierten Vorrichtung nach Anspruch 18, bei dem der Schritt des Änderns der Ausgabe der Tastausgabeeinheit das Abschalten der Tastausgabeeinheit umfasst.

20. Verfahren zum Orten einer implantierten Vorrichtung nach Anspruch 18, bei dem der Schritt des Änderns der Tastausgabeeinheit das Anschalten der Tastausgabeeinheit umfasst.

## Revendications

1. Système pour localiser un dispositif implantable comportant :
un dispositif implantable (4) ;
un émetteur (22) ;
un réseau d'antennes (3), le réseau ayant une première antenne réceptrice (30), une deuxième antenne réceptrice (31), une troisième antenne réceptrice (32), un processeur (2) relié aux antennes, le processeur (2) mesurant la quantité d'énergie provenant de l'émetteur (22) et détectée par chacune des antennes ; l'émetteur (22) ou le réseau (3) étant intégré dans ledit dispositif implantable, et également **caractérisé en ce que** le processeur (2) a un capteur d'égalité (51, 53, 54) pour détecter si l'énergie détectée par toutes les antennes (30, 31, 32) est égale.

2. Système selon la revendication 1, dans lequel le processeur (2) détecte par intermittence l'énergie reçue par chaque antenne (30, 31, 32).

3. Système selon la revendication 2, dans lequel le processeur (2) détecte par intermittence l'énergie reçue par chaque antenne pendant des quantités de temps égales pour chaque antenne.

4. Système selon une quelconque revendication précédente, dans lequel le processeur (2) a un capteur à seuil prédéterminé (51) pour détecter si l'énergie détectée par chacune des antennes dépasse un seuil prédéterminé.

5. Système selon une quelconque revendication précédente, dans lequel le réseau (3) comporte en outre des moyens (200, 201, 202, 203, 204, 205) pour indiquer la direction de l'émetteur (22) par rapport au réseau (3).

6. Système selon la revendication 5, dans lequel les moyens (200, 201, 202, 203, 204, 205) pour indiquer la direction de l'émetteur (22) par rapport au réseau (3) sont reliés au processeur (2), le processeur ayant des moyens pour activer sélectivement les moyens d'indication.

7. Système selon la revendication 5 ou 6, dans lequel les moyens d'indication comportent une série de flèches lumineuses (200, 201, 202, 203, 204, 205).

8. Système selon la revendication 7, dans lequel les flèches lumineuses (200 à 205) comportent une partie de queue et une partie de tête, la partie de tête pouvant être éclairée séparément de la partie de queue.

9. Système selon l'une quelconque des revendications précédentes, dans lequel le dispositif implantable comporte un dispositif de fourniture de médicament (4), le dispositif de fourniture de médicament ayant un réservoir de médicament (10), le réservoir de médicament ayant un septum de réservoir de médicament (11) à travers laquelle le réservoir de médicament peut être réapprovisionné.

10. Procédé de localisation d'un dispositif implanté (4) en utilisant un système de localisation tel que revendiqué dans une quelconque revendication précédente, comportant les étapes consistant à :
émettre de l'énergie à partir de l'émetteur (22) ;
déplacer le réseau d'antennes (3) le long de la surface du patient ;
détecter l'énergie reçue par chacune des antennes du réseau d'antennes ; et
émettre, en fonction de l'énergie détectée par chacune des antennes, un signal de localisation de dispositif vers l'opérateur du réseau.

11. Procédé de localisation d'un dispositif implanté selon la revendication 10, comportant en outre les étapes consistant à comparer si l'énergie reçue par chacune des antennes est la même, et à émettre ledit signal de localisation de dispositif lors de la détection du fait que l'énergie détectée par chacune des antennes est la même.

12. Procédé de localisation d'un dispositif implanté selon la revendication 10 ou 11, dans lequel l'étape d'émission d'un signal de localisation de dispositif comporte l'émission d'un signal lumineux.

13. Procédé de localisation d'un dispositif implanté selon la revendication 10, 11 ou 12, dans lequel l'étape d'émission d'énergie à partir de l'émetteur (22) est déclenchée lors de la réception d'un signal de télémétrie par le dispositif implanté (4).

14. Procédé de localisation d'un dispositif implanté selon l'une quelconque des revendications 10 à 13, dans lequel l'étape d'émission d'énergie à partir de l'émetteur comporte l'activation d'une bobine d'implant (22) dans le dispositif implanté pour émettre de l'énergie à une fréquence connue.

15. Procédé de localisation d'un dispositif implanté selon l'une quelconque des revendications 10 à 14, dans lequel l'étape de détection de l'énergie reçue par chacune des antennes (30, 31, 32) du réseau d'antennes (3) comporte en outre à déterminer si l'énergie reçue par chacune des antennes est supérieure à un minimum prédéterminé.

16. Procédé de localisation d'un dispositif implanté selon la revendication 15, comportant de plus l'étape consistant à émettre un signal de plage de fonctionnement en fonction du résultat de ladite étape de détermination.

17. Procédé de localisation d'un dispositif implanté selon l'une quelconque des revendications 10 à 16, dans lequel l'étape d'émission d'un signal de localisation de dispositif comporte l'émission d'un son.

18. Procédé de localisation d'un dispositif implanté selon l'une quelconque des revendications 10 à 17, comportant également l'étape consistant à prévoir un élément de sortie tactile pour le réseau ; l'étape d'émission d'un signal de localisation de dispositif comportant en outre la modification de la sortie de l'élément de sortie tactile.

19. Procédé de localisation d'un dispositif implanté selon la revendication 18, dans lequel l'étape de modification de la sortie de l'élément de sortie tactile comporte la désactivation de l'élément de sortie tactile.

20. Procédé de localisation d'un dispositif implanté selon la revendication 18, dans lequel l'étape de modification de la sortie de l'élément de sortie tactile comporte l'activation de l'élément de sortie tactile.
